# EUROPÄISCHE PATENTANMELDUNG

(11) **EP 0 806 405 A1**
(43) Veröffentlichungstag der Anmeldung: **12.11.1997**
(21) Anmeldenummer: 97107263.2
(22) Anmeldetag: 02.05.1997
(51) Int. Cl.: C07C 49/04, C07C 47/21, C07C 43/303, C07C 45/62, C07C 45/74, C07C 45/51, C07C 41/48, C07C 41/54

(54) **Verfahren zur Herstellung von Hexahydrofarnesylaceton aus 6.7-Dihydro-geraniol sowie neue Zwischenprodukte für dieses Verfahren**

(30) Priorität: 10.05.1996 DE 19619013
(71) Anmelder: BASF AKTIENGESELLSCHAFT, 67056 Ludwigshafen (DE)
(72) Erfinder: Teles, Joaquim Henrique, 67059 Ludwigshafen (DE); Hoffmann, Werner, Dr., 67141 Neuhofer (DE)

(57) **Zusammenfassung**

Verfahren zur Herstellung von Hexahydrofarnesylaceton der Formel I 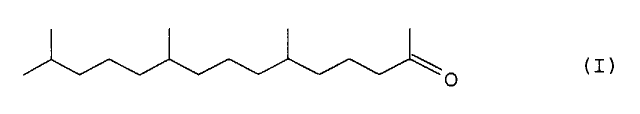 das dadurch gekennzeichnet ist, daß man
A. 6,7-Dihydro-geraniol der Formel II 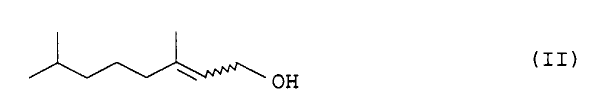 in Gegenwart von sauren Katalysatoren
   a) mit einem Prenaldialkylacetal der allgemeinen Formel III oder
   b) mit einem 1-Alkoxy-3-methyl-butadien der Formel IV 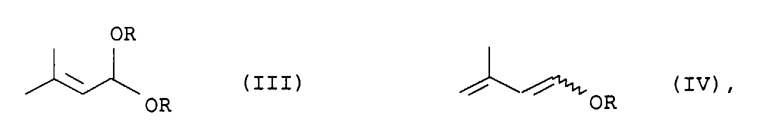
   in denen R für C₁- bis C₄-Alkyl, vorzugsweise Methyl, steht, auf Temperaturen von 50 bis 120°C zu den neuen Verbindungen 2,8,12-Trimethyl-4-alkoxy-5-oxa-trideca-2,7-dien der allgemeinen Formel V 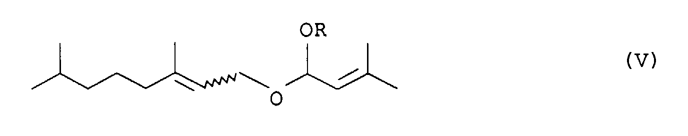 umsetzt,
B. diese in Gegenwart von sauren Katalysatoren unter Abspaltung von Alkanol in das neue 10,11-Dihydro-farnesal der Formel VI überführt 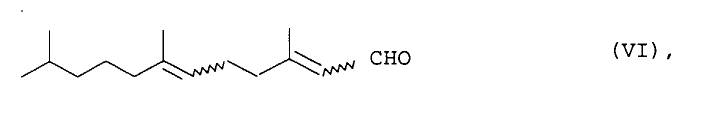
C. dieses in einer Claisen-Schmitt-Reaktion mit Aceton zu 13,14-Dihydro-3,4-dehydro-farnesylaceton der Formel VII kondensiert 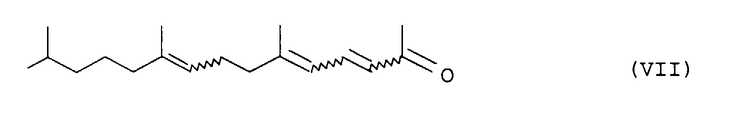
und D. dieses in an sich bekannter Weise katalytisch hydriert.

## Beschreibung

Die Erfindung betrifft ein verbessertes Verfahren zur Herstellung von Hexahydrofarnesylaceton sowie 2,8, 12-Trimethyl-4-alkoxy-5-oxa-trideca-2,7-dien und 10,11-Dihydro-farnesal als neue Zwischenprodukte für dieses Verfahren.

Hexahydrofarnesylaceton (6,10,14-Trimethyl-penta-decan-2-on) ist ein wichtiges Zwischenprodukt für die Herstellung von Phytol bzw. Isophytol und damit für die Herstellung von Vitamin E.

Technisch wird Hexahydrofarnesylaceton bisher im wesentlichen ausgehend von 6,7-Dihydro-linalool oder 1,2-Dehydro-linalool durch sequentielle Addition von einem C₃-Baustein, einem C₂-Baustein und wieder einem C₃-Baustein hergestellt. Beispielsweise kann es auf folgenden Synthesewegen hergestellt werden (vgl. Ullmann's Encyclopedia of Industrial Chemistry, Vol. A 27, Seiten 478-488, insbesondere 485-486)

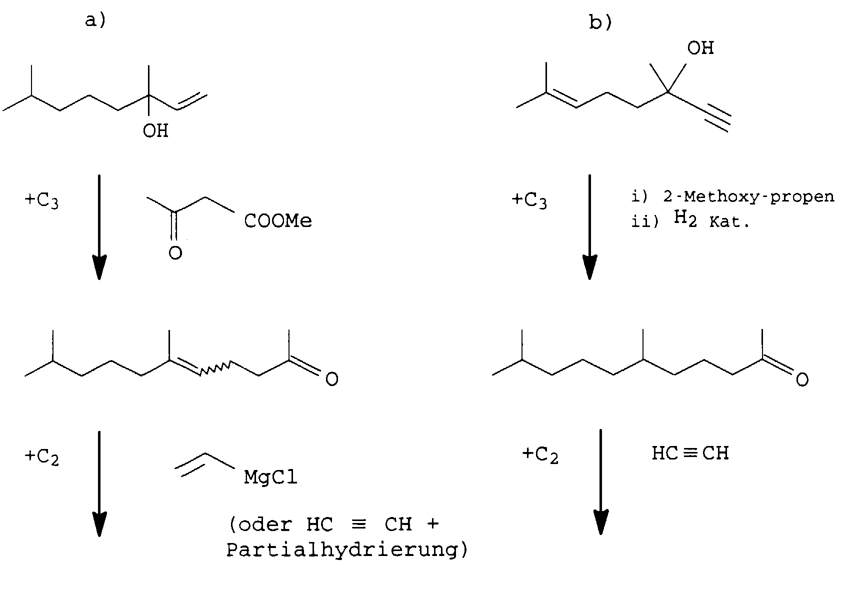

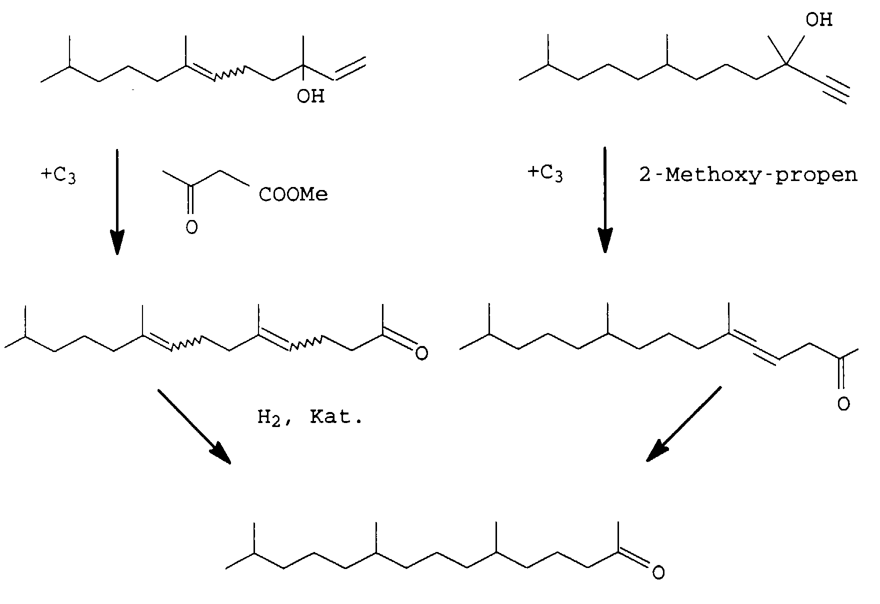

Nachteilig an Syntheseweg a) ist, daß er über 4 oder 5 Reaktionsstufen verläuft und daß man mit den teuren und schwierig handhabbaren Reaktanten Vinylmagnesiumhalogeniden oder Acetylen arbeiten muß. Nachteilig an Syntheseweg b) ist, daß er über 4 oder 5 Reaktionsstufen verläuft und daß man mit Acetylen und mit dem Isopropenylmethylether (2-Methoxy-propen) arbeiten muß.

Es war daher die Aufgabe der Erfindung, einen Syntheseweg zu finden, der über weniger Reaktionsstufen verläuft und bei dem man mit billigeren und weniger gefährlichen Reaktionspartnern arbeiten kann.

Gegenstand der Erfindung ist nun ein Verfahren zur Herstellung von Hexahydrofarnesylaceton der Formel I 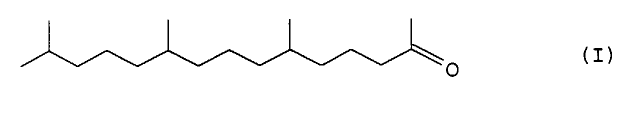 das dadurch gekennzeichnet ist, daß man
A. 6.7-Dihydro-geraniol der Formel II 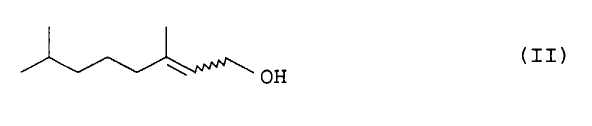 in Gegenwart von sauren Katalysatoren mit einem 3-Methyl-2-butenaldialkylacetal der allgemeinen Formel III oder mit einem 1-Alkoxy-3-methyl-butadien der Formel IV 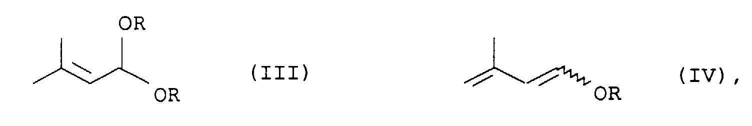 in denen R für C₁- bis C₄-Alkyl, vorzugsweise Methyl steht, bei Temperaturen von 50 bis 120°C, vorzugsweise 80 bis 110°C zu der neuen Verbindung 2,8,12-Trimethyl-4-alkoxy-5-oxa-trideca-2,7-dien der allgemeinen Formel V 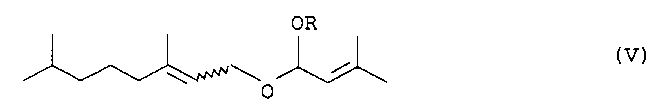 umsetzt,
B. diese neue Verbindung der Formel V in Gegenwart von sauren Katalysatoren unter Abspaltung von Alkanol der Formel ROH auf Temperaturen von 120 bis 250, vorzugsweise 125 bis 170°C erhitzt,
C. das dabei erhaltene neue 10,11-Dihydro-farnesal der Formel VI 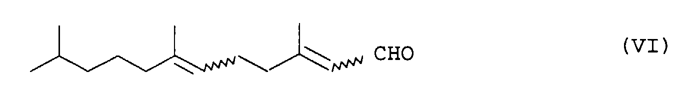 in einer Claisen-Schmitt-Reaktion mit Aceton zu 13,14-Dihydro-3,4-dehydro-farnesylaceton der Formel VII kondensiert 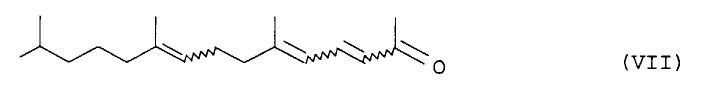
und D. dieses 13,14-Dihydro-3,4-dehydro-farnesyl-aceton der Formel VII in an sich bekannter Weise katalytisch hydriert.

Gegenstand der Erfindung sind außerdem 2,8,12-Trimethyl-4-alkoxy-5-oxa-trideca-2,7-diene der allgemeinen Formel V und 10,11-Dihydro-farnesal der Formel VI als Zwischenprodukte für dieses Verfahren.

Besonders vorteilhaft gestaltet sich das erfindungsgemäße Verfahren, wenn man im Reaktionsschritt A. das 6,7-Dihydro-geraniol mit 3-Methyl-2-butenaldimethylacetal (III mit R¹ = -CH₃) oder mit 1-Methoxy-3-methyl-butadien (VI mit R¹ = -CH₃) in 2,8,12-Trimethyl-4-methoxy-5-oxa-trideca-2,7-dien (V mit R¹ = -CH₃) umsetzt.

Das als Ausgangsverbindung verwendete 6,7-Dihydro-geraniol ist eine bekannte Verbindung und kann beispielsweise durch Umlagerung von 6,7-Dihydro-linalool hergestellt werden.

Als Dialkylacetal von 3-Methyl-2-butenal (Prenal) der allgemeinen Formel III in Reaktionsstufe Aa. verwendet man bevorzugt Prenaldimethylacetal oder Prenaldiethylacetal. Die Verwendung von Prenaldialkylacetalen mit unterschiedlichen Alkoholresten, wie Prenalmethylethyldiacetal ist prinzipiell möglich, aber nicht besonders vorteilhaft, da diese schwieriger herstellbar sind und zudem ein Gemisch von 2 verschiedenen 4-Alkoxy-verbindungen der Formel V gebildet werden kann.

Zur Durchführung der Reaktionsstufe Aa werden die Reaktanten der Formeln II und III im allgemeinen in Gegenwart eines sauren Katalysators unter Abdestillieren von 1 Mol des im Prenaldialkylacetal enthaltenen Alkanols auf Temperaturen bis zu 120°C erhitzt.

Als saure Katalysatoren können alle üblicherweise als saure Katalysatoren einsetzbaren sauren Verbindungen verwendet werden. Genannt seien beispielsweise anorganische Säuren, wie HCl, HNO₃, H₂SO₄, HClO₄ oder H₃PO₄; organische Säuren, z.B. aliphatische Carbonsäuren, wie Ameisensäure, Essigsäure, Propionsäure, Buttersäure, Chloressigsäuren, Oxalsäure, Weinsäure; aromatische Carbonsäuren, wie Benzoesäure, Toluylsäuren, Phthalsäure, Terephthalsäure oder Halogenbenzoesäuren; aliphatische, cycloaliphatische oder aromatische Sulfonsäuren, wie Methansulfonsäure, Cyclohexansulfonsäure, Benzolsulfonsäure oder p-Toluolsulfonsäure; feste saure Katalysatoren, wie SiO₂, Al₂O₃, TiO₂ oder Gemische dieser Oxide sowie Salze einer starken Säure und einer schwach basischen Substanz, wie Ammoniumchlorid, -nitrat, -sulfate, -phosphate oder saure Ionenaustauscher.

Unter den genannten sauren Katalysatoren werden stark saure Verbindungen, wie HCl, HNO₃, H₂SO₄, Cl₃CCOOH, Methansulfonsäure, Cyclohexansulfonsäure, Benzolsulfonsäure oder p-Toluolsulfonsäure bevorzugt.

Den sauren Katalysator verwendet man im allgemeinen in Mengen von 10⁻⁶ bis 1, vorzugsweise 10⁻⁴ bis 10⁻² Mol pro Mol Prenaldialkylacetal.

Die Prenaldialkylacetale und 6,7-Dihydro-geraniol verwendet man im allgemeinen in einem Molverhältnis von 1 bis 5, vorzugsweise 1,5 bis 3 Mol Diacetal der Formel III pro Mol 6,7-Dihydro-geraniol.

Ähnliche Umsetzungen von Allylalkoholen mit Prenaldialkylacetalen sind aus DE 24 39 140 bekannt.

Als 1-Alkoxy-3-methyl-butadien der allgemeinen Formel IV in Reaktionsstufe Ab verwendet man bevorzugt 1-Methoxy- oder 1-Ethoxy-3-methyl-butadien, insbesondere 1-Methoxy-3-methyl-butadien. Diese Verbindungen bilden sich in schwach saurem Milieu teilweise schon bei Raumtemperatur durch Alkoholabspaltung aus den Prenaldialkylacetalen der allgemeinen Formel III, so daß man vermuten kann, daß auch die Umsetzung der Prenaldialkylacetale mit dem 6,7-Dihydro-geraniol - zumindest teilweise - über die 1-Alkoxy-3-methyl-butadiene der Formel IV verläuft.

Die Reaktionsbedingungen für diese Umsetzung sind daher im wesentlichen dieselben wie sie für die Umsetzung mit Prenaldialkylacetalen beschrieben wurden.

Eine Umsetzung von wahlweise Prenaldialkylacetalen oder 1-Alkoxy-3-methyl-butadienen wurde schon in JP-A-0050/301 Kuraray; 07.09.73 (CA 84:5203) beschrieben, hier wurde jedoch ein Gemisch aus Hg(OAc)₂ und NaOAc als Katalysator verwendet.

Die neuen 2,8,12-Trimethyl-4-alkoxy-5-oxa-trideca-2,7-diene können nach Neutralisieren des Reaktionsgemisches unter stark vermindertem Druck destilliert werden. 2,8,12-Trimethyl-4-methoxy-5-oxa-trideca-2,7-dien destilliert unter teilweiser Zersetzung bei 97 bis 108°C/0,1 mbar als Isomerengemisch.

Zur Durchführung der Reaktionsstufe B. wird das 2,8,12-Trimethyl-4-alkoxy-5-oxa-trideca-2,7-dien entweder in isolierter und destillierter Form oder aber ohne Zwischenisolierung in Gegenwart saurer Katalysatoren unter Abdestillieren von Alkanol auf Temperaturen von 120 bis 250°C, vorzugsweise 125 bis 170°C erhitzt. Als saure Katalysatoren können die für Reaktionsstufen Aa. und Ab. genannten Katalysatoren verwendet werden.

Man verwendet die Katalysatoren in Mengen von 10⁻⁶ bis 1, vorzugsweise 10⁻⁴ bis 10⁻² Mol Säure pro Mol 2,8,12-Trimethyl-4-alkoxy-5-oxa-trideca-2,7-dien.

Arbeitet man ohne Zwischenisolierung der 4-Alkoxy-Verbindung der Formel V, so braucht dem Reaktionsgemisch in aller Regel kein neuer saurer Katalysator zugesetzt werden.

Das in Reaktionsstufe B. erhaltene neue 10,11-Dihydro-farnesal der Formel VI kann nach Neutralisation unter stark vermindertem Druck destilliert werden. Es destilliert bei 128 - 132°C/0,5 mbar in Form einer blaßgelben, angenehm riechenden Flüssigkeit als Isomerengemisch.

Ähnliche Reaktionen zur Herstellung von Citral sind aus DE 24 39 140 und DE 24 11 530 bekannt.

Zur Durchführung von Reaktionsstufe C. wird das erhaltene neue 10,11-Dihydro-farnesal in einer Claisen-Schmitt-Reaktion mit Aceton kondensiert.

Bezüglich näherer Einzelheiten über die Reaktionsbedingungen bei Claisen-Schmitt-Reaktionen verweisen wir auf Org.-Synth.-, Coll. Vol. 3, Seiten 747-750.

Das so erhaltene 13,14-Dihydro-3,4-dehydro-farnesylaceton kann in an sich bekannter Weise durch katalytische Hydrierung in das gewünschte Hexahydrofarnesylaceton überführt werden. Bezüglich näherer Einzelheiten über katalytische Hydrierungen von Polyenen verweisen wir beispielsweise auf EP 34 804.

Mit besonderem Vorteil führt man das erfindungsgemäße Verfahren ohne Zwischenisolierung der neuen 2,8,12-Trimethyl-4-alkoxy-5-oxa-trideca-2,7-diene der allgemeinen Formel V durch.

Mit Hilfe des erfindungsgemäßen Syntheseweges kann man das begehrte Hexahydrofarnesylaceton in nur 3 Reaktionsstufen unter Vermeidung von schwer handhabbaren Reaktanten, wie Acetylen und Vinylmagnesiumchlorid herstellen.

### Beispiel 1

A. Herstellung von 2,8,12-Trimethyl-4-methoxy-5-oxa-trideca-2,7-dien aus 6,7-Dihydro-geraniol
   26,0 g (0,17 mol) 6,7-Dihydro-geraniol, 32,5 g (0,25 mol) Prenaldimethylacetal und 0,5 g (0,0025 mol) para-Toluolsulfonsäure wurden in einem 250 ml 3-Halskolben mit Vigreux-Kolonne und Destillationsaufsatz vorgelegt und die Temperatur langsam bis auf 100°C erhöht, wobei Methanol abdestillierte. Die Reaktion wurde solange forgesetzt bis kein Methanol mehr überdestillierte (insgesamt ca. 5 g entsprechend 0,16 mol). Nach dem Abkühlen wurde mit festem Natriumhydrogencarbonat neutralisiert und unter vermindertem Druck destilliert. Das Produkt destillierte bei 0,1 mbar mit teilweiser Zersetzung bei 97 - 108°C als Isomerengemisch. Die Ausbeute betrug 24,7 g (60 % der Theorie).

### Beispiel 2

A. und B. Herstellung von 10,11-Dihydro-farnesal aus 6,7-Dihydro-geraniol ohne Zwischenisolierung von 2,8,12-Trimethyl-4-methoxy-5-oxa-trideca-2,7-dien
   50 g (0,32 mol) 6,7-Dihydro-geraniol 62,4 g (0,48 mol) Prenaldimethylacetal und 1,0 g (0,005 mol) para-Toluolsulfonsäure wurden in einem 250 ml 3-Halskolben mit Vigreux-Kolonne und Destillationsaufsatz vorgelegt und die Temperatur langsam bis auf 90°C erhöht, wobei Methanol abdestillierte. Die Reaktion wurde solange fortgesetzt bis kein Methanol mehr überdestillierte. Die Temperatur wurde dann auf 150°C erhöht, wobei erneut Methanol überdestillierte. Als kein Methanol mehr überdestillierte wurde der Ansatz abgekühlt, mit festem Natriumhydrogencarbonat neutralisiert und unter vermindertem Druck destilliert. Das Produkt destillierte bei 128 - 132°C bei 0,5 mbar als Isomerengemisch in Form einer blaßgelben, angenehm riechenden Flüssigkeit. Die Ausbeute betrug 53,8 g (entsprechen 76 % der Theorie).
C. Herstellung von 13,14-Dihydro-3,4-dehydro-farnesylaceton aus 10,11-Dihydro-farnesal
   25 g (0,11 mol) 10,11-Dihydro-farnesal und 63,8 g (1,1 mol) Aceton wurden in einem Kolben vorgelegt. Unter Rühren wurde dann eine Lösung von 1,18 g (0,022 mol) Natriummethylat in 10 ml Ethanol bei 22°C innerhalb von 7 Minuten (min) zugetropft. Das Gemisch wurde weitere 5 min gerührt und dann in eine Lösung aus 0,75 g (0,005 mol) Weinsäure in 10 ml Ethanol gegossen. Der entstandene Niederschlag wurde abgenutscht und das Filtrat am Rotationsverdampfer eingeengt. Der Rückstand wurde mit 25 ml Wasser aufgeschlämmt und dreimal mit je 25 ml Diethylether extrahiert. Die organischen Phasen wurden gesammelt, über Natriumsulfat getrocknet und eingeengt. Der Rückstand (ca. 25 g) wurde unter verminderten Druck destilliert. Das Produkt siedete bei 163 - 170°C/0,1 mbar als gelbe Flüssigkeit. Die Ausbeute betrug 14,0 g entsprechend 49 % der Theorie.
D. Herstellung von Hexahydrofarnesylaceton aus 13,14-Dihydro-3,4-dehydro-farnesylaceton
   2,7 g (0,01 mol) 13,14-Dihydro-3,4-dehydro-farnesylaceton wurde bei 90°C und 10 bar Wasserstoffdruck hydriert bis kein Wasserstoff mehr aufgenommen wurde (ca. 24 Stunden). Als Katalysator wurden ca. 100 mg Palladium (0,5 Gew.-%) auf Aluminiumoxid verwendet. Nach Abtrennung des Katalysators durch Filtration wurde Hexahydrofarnesylaceton als farblose Flüssigkeit in fast quantitativer Ausbeute isoliert. GC-MS-Analytik des Produkts zeigt nur noch Spuren von einfach ungesättigten Verbindungen.

## Patentansprüche

1. Verfahren zur Herstellung von Hexahydrofarnesylaceton der Formel I 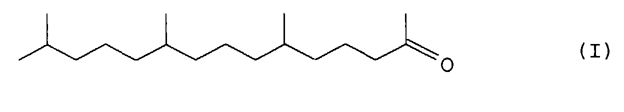 dadurch gekennzeichnet, daß man
A. 6,7-Dihydro-geraniol der Formel II 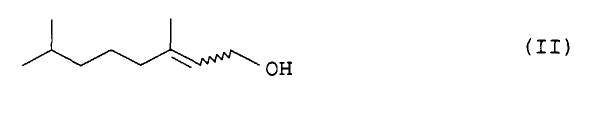 in Gegenwart eines sauren Katalysators
a) mit einem 3-Methyl-2-butenaldialkylacetal der allgemeinen Formel III oder
b) mit einem 1-Alkoxy-3-methyl-butadien der Formel IV 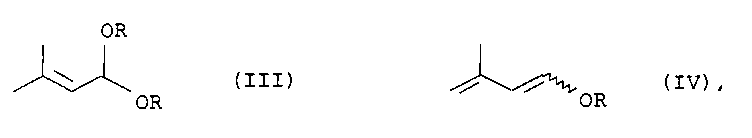
in denen R für C₁- bis C₄-Alkyl, vorzugsweise Methyl steht,
auf Temperaturen von 50 bis 120°C, vorzugsweise 80 bis 110°C zu der neuen Verbindung 2,8,12-Trimethyl-4-alkoxy-5-oxa-trideca-2,7-dien der allgemeinen Formel V 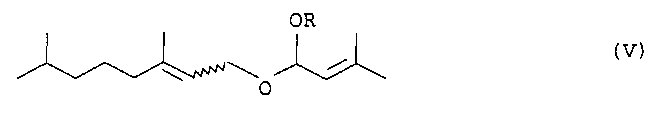 umsetzt,
B. die erhaltene neue Verbindung der Formel V in Gegenwart von sauren Katalysatoren unter Abspaltung von Alkanol der Formel ROH auf Temperaturen von 120 bis 250, vorzugsweise 125 bis 170°C erhitzt,
C. das dabei erhaltene neue 10,11-Dihydro-farnesal der Formel VI 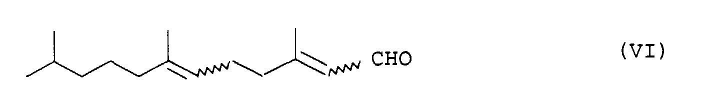 in einer Claisen-Schmitt-Reaktion mit Aceton zu 13,14-Dihydro-3,4-dehydro-farnesylaceton der Formel VII kondensiert 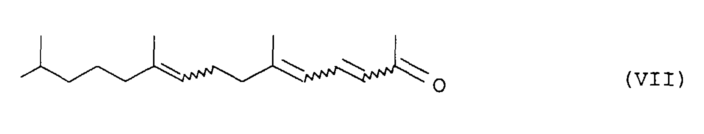
und D. das erhaltene 13,14-Dihydro-3,4-dehydro-farnesylaceton der Formel VII in an sich bekannter Weise katalytisch hydriert.

2. Verfahren gemäß Anspruch 1, dadurch gekennzeichnet, daß man im Reaktionsschritt A. das 6,7-Dihydro-geraniol mit Prenaldimethylacetal oder 1-Methoxy-3-methyl-butadien zu 2,8,12-Trimethyl-4-methoxy-5-oxa-trideca-2,7-dien umsetzt.

3. 2,8,12-Trimethyl-4-alkoxy-5-oxa-trideca-2,7-dien der allgemeinen Formel V 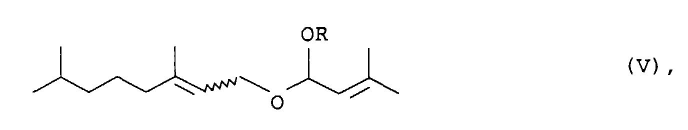 in der R für C₁- bis C₄-Alkyl, vorzugsweise Methyl- oder Ethyl steht.

4. Verfahren zur Herstellung von 2,8,12-Trimethyl-4-alkoxy-5-oxa-trideca-2,7-dien der allgemeinen Formel V, dadurch gekennzeichnet, daß man Hexahydrofarnesylaceton in Gegenwart eines sauren Katalysators
a) mit einem 3-Methyl-2-butenaldialkylacetal der allgemeinen Formel III, oder
b) mit einem 1-Alkoxy-3-methyl-butadien der allgemeinen Formel IV auf Temperaturen von 50 bis 120°C erhitzt.

5. 10,11-Dihydro-farnesal der Formel VI

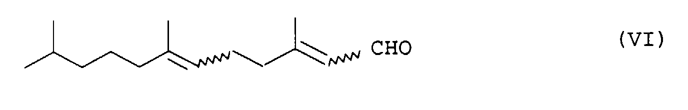

6. Verfahren zur Herstellung von 10,11-Dihydro-farnesal der Formel VI, dadurch gekennzeichnet, daß man 2,8,12-Trimethyl-4-alkoxy-5-oxa-trideca-2,7-diene der allgemeinen Formel V in Gegenwart von sauren Katalysatoren unter Abspaltung von ROH auf Temperaturen von 120 bis 250°C, vorzugsweise 125 bis 170°C erhitzt.

7. Verfahren gemäß Anspruch 1 zur Herstellung von Hexahydrofarnesylaceton der Formel I, dadurch gekennzeichnet, daß man das Verfahren ohne Zwischenisolierung der neuen 2,8,12-Trimethyl-4-alkoxy-5-oxa-trideca-2,7-diene der allgemeinen Formel V durchführt.
